# EUROPEAN PATENT APPLICATION

(11) **EP 3 888 682 A2**
(43) Date of publication of application: **06.10.2021**
(21) Application number: 19890735.4
(22) Date of filing: 25.11.2019
(51) Int. Cl.: A61K 45/00, A61K 33/00, A61K 31/00, A61P 19/02, A23L 33/16, A61P 37/02, A23L 33/10

(54) **SUBSTANCE FOR TREATING AND/OR PREVENTING DISEASES, AND DESIGN METHOD AND PREPARATION METHOD THEREFOR**

(30) Priority: 28.11.2018 CN 201811437559; 25.08.2019 CN 201910787173
(71) Applicant: Dong, Futian, Suzhou, Jiangsu 215123 (CN)
(72) Inventor: Dong, Futian, Suzhou, Jiangsu 215123 (CN)
(74) Representative: Habermann, Hruschka & Schnabel
(86) International application number: PCT/CN2019/120723
(87) International publication number: WO 2020/108444

(57) **Abstract**

The present disclosure provides a substance for treatment and/or prevention of diseases, the method for designing the substance, and the method for preparing the substance. A stable structure corresponding to a substance is used as the substance for treatment and/or prevention of diseases associated with the substance. The essence of disease is the imbalance of the biological structure system. Different structural imbalances will cause different diseases. The present invention discloses that the biological body recognizes the stable structure corresponding to the substance, adjusts its gene or gene expression by the self-adaptation and self-organization functions of its own structural system, and restores the biological structure system to be related to the substance. The balance of structure, treatment and/or prevention of diseases related to the substance.

## Description

### Technical field

The present disclosure relates to the technical field of disease treatment, medicaments, health foods, food additives and the like, and specifically relates to substances for treatment and/or prevention of diseases, the method for designing them, and the method for preparing them.

### Background Art

According to a new medical model-*Structural information medicine,* the present invention provides understanding of life from the perspective of the *structure system,* and achieves the purpose of preventing and treating diseases by allowing the body to recognize specific structural information, and utilizing function, such as the self-adapting and self-organizing of the structure system of the body to restore the dynamic balance of the structure system.

### 1. Medical models of traditional Chinese medicine and Western medicine

Western medicine and traditional Chinese medicine are major existing medical systems. Western medicine is based on microscopic local structures of substances, and has been doing well in characterizing substances, but not so well in formulating general laws underlying substances, whereas the traditional Chinese medicine is mainly based on a general overall functional system, and has been doing well in formulating general laws underlying substances but not in characterizing them. The two medical systems complement each other, but have theories and research methods opposite to each other. Western medicine mainly relies on scientific experimentation to analyze the material nature of diseases by reductionism, by means of basic research methods such as anatomical physiology, biochemistry, molecular biology, and clinical trials. Therefore, Western medicine pays more attention to the pathologically changed structural substances, analyzes specific changes in structure, and directly curbs the excessive and remedies the deficient at a level of local structure of substances by means of chemical drugs, surgeries, radiation, or the like. For example, therapies such as H2 receptor antagonists for treatment of gastric hyperacidity and hormone replacement therapy take effect rapidly and have been highly effective in treatment of some acute diseases, surgical diseases and infectious diseases. However, since comprehensive understanding of the entire body has not been achieved in Western medicine, such therapies can only temporarily solve problems with a local structure, and long-term application thereof will have an adverse effect on other structures of the body and disrupt the balance in the body. In fact, since 2010, one third of diseases in the world are drug-induced diseases, which are caused by inevitable side effects of drugs. In addition, Western medicine has a mild effect on chronic degenerative diseases caused by complex pathogenic factors and influenced by multiple factors, such as cardiovascular and cerebrovascular diseases, multiple sclerosis, and senile dementia, and has difficulty in curbing the increasing morbidity and mortality due to these diseases.

Traditional Chinese medicine treats life as a whole, unifies people, society, psychology and environment, and reflects the objective laws of nature and people as well as health and disease, with *Bianzheng and Lunzhi* (identification of indication then treatment) as its central principle. In traditional Chinese medicine, the concept "health" refers to a relatively balanced, coordinated, and ordered state of the body's *Yin* and *Yang* in both subjective and objective environments, that is, the balance of the functional system. When this balance is disrupted, the order and homeostasis in the human body is disturbed and damaged, that is, the functional system of the human body is out of balance, and then diseases appear. Traditional Chinese medicine diagnoses the nature of diseases by consulting patients for a large amount of information about the functional system, and then promotes the body's functional system by means of drugs, acupuncture, massage, and the like. In general, it curbs the excessive, remedies the deficient, strengthens the body resistance (positive, ZHENG, or GOOD), eliminates the pathology (negative, XIE, or BAD), and promotes the positives, thereby restoring the homeostasis of the body. Traditional Chinese medicine takes effect slowly, but reduces the side effects of replacement therapies or antagonistic drugs, and has been quite effective in prevention and treatment of diseases, health preserving, and rehabilitation.

In summary, Western medicine focuses more on the microscopic structural balance, while traditional Chinese medicine pays more attention to the macroscopic functional system balance, both of which aim to correct the imbalance of the body but at different levels. However, they are completely different in epistemology and methodology about diseases and health. In order to take the benefits of both medical models, we need to integrate them with a more inclusive perspective and theory, so as to develop substances more effective in treatment of diseases and to significantly benefit human health.

### 2. Structural information medicine

### (1) A fundamental theory that integrates the Western medicine and traditional Chinese medicine with the structure system

After the *Big Bang,* positive and negative charges appeared and interacted to form a substance of a simple structure, hydrogen, and then the interaction between the structures led to more complex structures, and finally a macroscopic substance, including the entire biological world. Therefore, all things in the universe are ascribed to the structures, and the substance, energy, and information that constitute the universe are also ascribed to the structures. Firstly, different substances are made of different structures, and substances are specific structures and the basis for functioning. The structure and function of a living system match each other, a specific structure corresponds to a specific function, and a change in specific structure will also naturally affect the specific functions of the corresponding system. Secondly, the bonding energy of the interaction between structures is a relatively abstract structure indicating stability of structure, and energy is the internal driving force for interconnection of structures. Finally, the relationship and order between structures is information, and information is the most abstract structure among the three, combines and governs substances and energy, and reflects the internal law and nature of structures. For example, the nature of an organism is determined by its abstract structure-genes. Therefore, structure is the unification of substances, energy and information, that is, the unification of concrete structures and abstract structures.

An organism is an organic system, essentially an organic structure system dominated by abstract structures. This structure system is an organic structural whole with a certain function formed by a relatively stable communication mode, the order of organization, and the temporal and spatial relationships between a number of elements. Any element itself is also a structure system, a sub-system that constitutes a part of the original system and certainly consists of sub-sub-systems and so on. The biological structure system is composed of structural sub-systems at different levels, such as the immune system and the gastrointestinal system. On the other hand, an organism is a sub-system of a larger structure system. For example, an organism is a sub-system of nature-a larger structure system, and maintains its own existence and reproduction in constant exchanges of substance, energy and information with nature.

DNA as a genetic material of organisms stores all information in the process of life activities, including growth, development, aging, and death. According to the *biochemical individuality* theory by Dr. Roger Williams, difference in biochemical composition between human bodies originates from the difference in genes and gene expressions, that is, from the difference in the molecular spatial structure of gene expression. Dr. Susumu Tonegawa, a Nobel laureate in physiology, stated that all diseases except trauma are associated with genes. The causes of diseases mainly include two aspects: (i) a structure constituting a part of the body is damaged or changed by the environment of the structure, or (ii) a gene is improperly expressed. The root cause of structural imbalance is a change in an abstract structure, that is, a change in gene expression, such as abnormal histone acetylation and methylation, abnormal gene methylation, etc. If a specific structure is changed, as long as the genes related to the structure can be expressed normally, the body's metabolism will gradually replace the pathologically changed structure with a normal structure, so that the body can return to a healthy state. Therefore, the key to the health of an organism lies in genes and gene expressions.

Western medicine studies the changes in specific structures, building models from diseases and using the models to treat diseases. Western medicine focuses more on studies of local tissue and functions in the structure system. With the assistance of instrument, detection can be made deep into specific and microscopic structures of substances. The form of existence of specific structures and morphologies of substances in the human body are given more attention and weight, such as anatomy, molecular biology, cell biology, histology and embryology, etc. These disciplines experimentally investigate and observe specific structures in the structure system from different angles, but have not done enough in abstract thinking and have overlooked the integrity of the body as a structure system. Therefore, it is easy to have limitations in understanding and practice. Western medicine mainly directly intervenes in specific structures by changing and influencing the local structure in the body with drugs to achieve a new balance, including research on genes, which also tends to directly change genes or gene expressions. Traditional Chinese medicine builds models from humans, and studies the functions of various organs of the body and the relationships and interactions between the functions under the guidance of holism. Traditional Chinese medicine does not detect specific structures of substances, but diagnoses diseases through manifestations of functions and external information. However, functions are determined by structures, and the functional system is essentially a structure system. The mutual promoting and restraining effects between functional sub-systems are essentially the mutual enhancing and inhibiting actions between the structural sub-systems. Traditional Chinese medicine achieves an overall balance by regulating the functional order and strength of the body, the essence of which is to restore the dynamic balance of the structure system. However, due to lack of an in-depth understanding of specific structures in the body, traditional Chinese medicine also has great limitations. Western medicine mainly establishes disease models from the perspective of microscopic structures, while traditional Chinese medicine establishes life models from the perspective of macroscopic functions. The microscopic structures found by Western medicine provide a material basis for indications of traditional Chinese medicine, that is, all the indications of traditional Chinese medicine have a material support from gene expression, i.e., a structural support, and are essentially due to a microscopic structural imbalance. In the structure system, based on the unification of the microscopic and the macroscopic, parts and a whole, and the concrete and the abstract, the specific structures may be converted into abstract structural information by utilization of local and microscopic concrete structures and recognition of the concrete structures by the body, thereby stimulating the body's self-adapting and self-organizing function to regulate genes or gene expressions and regain the balance of the structure system.

As a system, the structure system has basic characteristics such as system integrity, openness, stability, and self-organization. Relative to the environment, the system is unification of enclosures and openness. In the structure system, the interaction among structures is based on the interaction between the structures and the environment as a proviso, and on the external environment of the structures as a condition. The evolution of the same structure system depends on the external structure. Proper changes and adjustments of gene expressions according to changes in the external structure is a fundamental function of an organism to adapt to the environment, maintain its survival, and maintain its own health. If this function is absent, it will generally become extinct. The structure system of the human body has this powerful health function. By structural information of specific structures, Structural information medicine aims to mobilize and modulate the relatively complex, highly ordered, and self-regulated mechanism of action in the structure system, including sub-systems at different levels, such as genes, molecules, cells, tissues and organs, so as to restore the body's structural balance and achieve the purpose of preventing and treating diseases. It not only integrates the Western medicine's theory focusing on specific structures and functions as research objects, but also integrates traditional Chinese medicine's theory focusing on restoration of balance by regulating the body itself.

### (2) Problems to be solved by Structural information medicine

Living organisms cannot exist independent of the environment. The external structural environment interacts with the body's structure system through structures, namely substance, energy and information. Each individual is a unique structure system resulting from both genes and environment. Structural information medicine focuses on the dynamic balance between the internal structure system in an organism and its external structural environment. It is this dynamic balance that interprets the process of life. In addition to restoring the dynamic balance of the body's own structure system and enhancing the body's resistance, structural information medicine further gives weight to symbiosis and co-progress. Symbiosis means that the body no longer blindly combats and defends itself against external structures, but constantly internalizes the external structural information, and achieves a balance between the its structure system and the external structure through the self-adapting and self-organizing function of the body, thereby turn the enemy into a friend, even a utilizable source. Co-progress means that the biological structure system obtains, through structural information, the structural characteristics from other organisms that are beneficial to health, survival and reproduction of organisms.

### Summary of Invention

The objective of the present disclosure is to provide a method for designing a substance for treatment and/or prevention of diseases to address the above-mentioned defects in the prior art.

Another objective of the present disclosure is to provide a substances designed by the above method.

Yet another objective of the present disclosure is to provide a method for preparation and use of the substances.

The biological structure system is an organic structure system with unified stability and adaptability. It can adapt to the environment, maintain the dynamic balance between itself and the external environment, and in turn achieve better survival and reproduction for itself. The adaptability of the biological structure system is based on the stability of the structure of the organism, and the stable structure is a dominating structure that plays a key role.

The biological structure system is an organic giant system formed by spiral development of a core structure composed of concrete structures and abstract structures. The core structure of the biological structure system, that is, a unified complex of the specific structure and the abstract structure in the nucleic acid-protein structure system, is the carrier and expression system of genetic information, plays a dominating role in functioning of the biological structure system, while in the core structure it is the stable structure that plays a dominating role. At the atomic level, the abstract structure DNA and the concrete structure protein in the core structure are both biological macromolecules based on the carbon element. The outermost layer of a carbon atom has 4 electrons, and it is neither easy to lose electrons nor easy to obtain electrons. This structure favors the formation of 4 covalent bonds from a carbon atom. A covalent bond has high stability and is the strongest force between organic molecules. The single covalent bond mainly formed by carbon atoms is about 300-500 KJ/moL, and the thermal breaking temperature is 500-800°C.

The primary structure of DNA determines the double helix secondary structure of DNA through hydrogen bonding and stacking force between bases. That is, the stable primary structure determines its adaptive secondary structure, and the core of genetic information is in the stable structure. DNA transfers the genetic information to RNA through transcription, and finally the genetic information from RNA is expressed into a protein through translation. This is the law followed by all cell-based organisms. Establishment of this central law clarifies the transfer path of genetic information from DNA to RNA to protein, i.e. from the base sequence in the primary structure of DNA to the base sequence in the primary structure of RNA to the amino acid sequence in the primary structure of protein. Through base complementary pairing and triplet codons, the genetic information carrier linearly maps pieces of information one by one to the functional information carrier-amino acids. Therefore, this information transfer path is based on the stable structure of these biological macromolecules, i.e. transfer from the stable structure of the abstract structure to the stable structure of the specific structure.

The covalent backbone of the primary structure of a protein contains hundreds of single bonds which are freely rotatable, but the freedom of rotation of each single bond is restricted by factors such as the rigid planar nature of the peptide bond, the number and position of hydrophilic and hydrophobic amino acids and acidic and basic amino acids in the primary structure, finally forming the thermodynamically most stable three-dimensional structure. Therefore, in fact, the factor controlling the final structure of a protein, that is, the amino acid sequence, is formed very early. These stable structures that govern the protein conformation form a framework, according to which the polypeptide chain assembles into the final form during folding. The amino acid sequence of a protein contains all the information that determines its three-dimensional structure, and the structure of the protein determines its function. Therefore, consistent with the genetic information, the functional information also has the nature that the stable structure determines the adapted structure.

From the perspective of a system, the stability of the biological structure system is a self-stabilizing ability of the organic structure system formed via structural interactions. Each structure in the structure system is promoted by some structures and inhibited by other structures, and they form a dynamic balance via interactions that is neither deficient nor excessive and can adjust itself within a certain range according to environmental changes, to maintain and restore the original ordered state, that is, to maintain its own order from an imbalanced state, in which the interactions between the stable structures play a dominating role. For example, for the recognition of structural information, the innate immune system in animals and plants can recognize structural characteristics of pathogens through pattern recognition receptors (PRRs), and these characteristics are stable molecular structures shared by different types of pathogens and do not easily mutate (for example, all bacteria have a cell wall comprising LPS). For another example, as regards food allergies, even if food containing allergens is steamed or boiled at a high temperature, it still causes allergies as the immune recognition is directed to the stable structure of the allergens. Taking neurotransmitter opioid peptides as an example, opioid peptides in different conformations have different selectivity for cell membrane receptors. Endomorphin-1 is a biologically active peptide known to have the highest affinity and selectivity to µ receptors. An adaptive structure is the molecular basis for the binding of signal molecules to receptors. From the perspective of variations in the stable structure amino acids, endomorphin-1 differs from other opioid peptides mainly in three amino acids. By the change in amino acid residues, it forms an appropriate spatial layout, changes its three-dimensional conformation, and provides correct positioning for binding of such transmitters to receptors. Interactions between the stable structures can cause significant changes in the adaptive structures, and play a crucial role in information recognition. During information transfer, receptors on the cell membrane need to bring the information to the nucleus, which mainly relies on modifications of proteins. The basis of protein modification is the chemical reactivity of amino acid residues in the primary structure. Under the action of enzymes, the stable structures of a protein can be adjusted to make the higher structure of the protein more adapted to the functional requirements under specific time and spatial conditions, with reversibility and versatility, for example phosphorylation and de-phosphorylation of proteins. To the information transferred to the nucleus, the cell eventually makes a response by altering the gene expression pattern, and in turn passes instructions down by signaling pathways. Therefore, interactions between the stable structures or adjustment of the structures determine the changes in the adaptive structures, and certainly the adaptive structures can also make a reaction on the stable structures.

In the biological structure system, no structure is "isolated". The interactions, mutual restriction and interdependence between structures and between structural sub-systems constitute the organic biological structure system. All organisms are made of cells, and cells are the structural sub-systems of the large biological structure system. Depending on its complexity, an organism may be composed of hundreds, tens of thousands, or even hundreds of millions of cells that exert various specific functions. The cells divide the work and cooperate to build an ordered and controllable cell society. Maintenance of such a society depends not only on the material metabolism and energy metabolism of the cells, but also on the communication and signal regulation between the cells, so that the behaviors of the cells, such as cell growth, division, differentiation, apoptosis and other physiological functions, can be coordinated to realize a complete life activity process of a multicellular organism. The essence of intercellular communication and signal regulation is also interaction between structures which determine the behavior and fate of cells, including structural and functional differentiation, location, and fateful choices. The morphology and structure, life activity and position of cells in the body are all regulated and controlled by the body, local tissue, surrounding cells, and extracellular signaling molecules. For example, nerve cells, immune cells and endocrine cells are in social communication to participate in and maintain the homeostasis of the body. The interaction between structures in the biological structure system makes the cell capable of distinguishing itself from others, and distinguishing between allogenic cells and heterogenic homologous cells. The phenomenon of mutual identification and recognition among cells, as well as the molecular recognition of self and foreign substances, is called cell recognition. Based on the recognition, cells adhere to each other or establish a stable connection with a certain morphology and structure, thereby influencing and interacting with each other. Many important life activities are associated with the recognition ability of cells. Cell recognition is also a very important step in the process of cell development and differentiation. Cells form different types of tissue through recognition and adhesion. Body immunity is also in fact a phenomenon of cell recognition. White blood cells in the blood can recognize invading bacteria and engulf them, but never engulf own normal cells in the blood. This is cell recognition between different species. After clinic transplantation of allogeneic tissue, the body will reject the transplanted allogeneic tissue, which is caused by the cell recognition between allogeneic cells. In addition, many physiological and pathological processes such as blood coagulation, inflammatory reaction, thrombosis, infections by pathogenic microorganisms and even tumor cells metastasis, are associated with the complex mutual recognition and adhesion between the allogenic and the xenogenic as well as the effects induced thereby. Structure recognition is based on the interaction between structures, which the basis of the self-adaptive, self-organizing organic structure system formed in an organism.

The openness of a structure system is not only the openness of specific structures, but also the openness of relationships. The self-adapting and self-organizing ability of the structure system is mainly reflected in the stabilization of the system under external environmental stimuli. The biological structure system makes self-adjustments and self-organizations through transmission and processing of information, and constantly overcomes the uncertainty in the structure system to keep it in a dynamic balance. The exchange between the structure system and its environment is not only exchange of substance, but also exchange of energy or information. To maintain the homeostasis in a constantly changing environment, an organism needs the ability of cells to sense and respond to these changes. The ability of cells to receive and transmit information and execute instructions is the basis for the organism to respond to changes in internal and external environments. Proteins are the performers of various physiological activities in a cell (such as catalyzing chemical reactions and regulating gene expressions), and changes in the own state of proteins will also change their functioning. Therefore, organisms use protein molecules possessed by cells to build a signaling system. Because the shape of a protein molecule can be changed, the functions closely related to the shape of the protein molecule will also change accordingly, and one change in the state of the protein will change the state of the downstream molecules through interaction with downstream protein molecules, which in turn further change the state of more downstream molecules. Such a cascade of changes in state is the way information is transmitted in a cell. Moreover, proteins are biologically functional molecules that make a final response to the signal after the change in state, and become effectors. The effectors at the end of the information transmission chain are mostly transcription factors, which respond to signals by regulating expressions of genes.

Taking the immune system as an example, any external structure having entered the human body will be recognized and evaluated by the immune system. For example, when bacteria or viruses enter the human body, immune cells interact with the foreign structures to recognize their structural information, and then secrete different cytokines according to the different structural information and transmit the information to B lymphocytes and regulate the gene expression of B cells to synthesize IgG antibodies that bind the antigen structure to form a complex, which is eventually eliminated by macrophages to resist foreign invasion. When parasitic pathogens invade, immune cells interact with the foreign structure to recognize structural information that activates B cells and regulates gene expression to synthesize IgE antibodies. These antibodies bind mast cells and basophils to release toxic granule proteins and the like from the cells to kill the parasites.

In this immune response, the recognized structure determines the specific feedback of the body. Through the interaction between structures, specific structures turn into an abstract structure-structural information. Based on the recognition of the structural information, the body mobilizes multiple cells in the immune system to work coordinately, regulates gene expressions, and releases different active substances to adaptively act against foreign objects and achieve a new stable and harmonious structure system. Meanwhile the immune system interacts with other systems to exchange material, energy, and information, to realize coordination of different sub-systems in the structure system. It can be seen that there are structural information recognition and self-regulation in the biological structure system, which restore the balance of the structure system, and even establish a new structural balance mechanism with external structures.

### (2) Features of the present disclosure

According to the established theory system of *Structural information medicine,* the present disclosure uses a stable structure corresponding to a substance to act on the biological structure system, and allows the organism to regulate its genes or gene expressions through the self-adapting and self-organizing ability of its own structure system, so as to treat diseases associated with the substance.

In view of this, the present disclosure provides a substance for treatment and/or prevention of diseases, the method for designing the substance, and the method for preparing the substance. The specific embodiments thereof are as follows.

A method for designing a substance for treatment and/or prevention of diseases, comprising:
using a stable structure corresponding to a substance as the substance for treatment and/or prevention of diseases associated with the substance.

The stable structure corresponding to the substance refers to a structure of the substance that is present in a solid state under a high temperature of 500°C or more.

Preferably, the diseases associated with the substance include a diseases caused by some materials in the substance and/or diseases caused by changes in some materials in the substance.

A substance for treatment and/or prevention of diseases, designed according to the above method.

A method for preparing the above substance for treatment and/or prevention of diseases, wherein, the stable structure corresponding to the substance or to a substance containing the substance is prepared by high-temperature carbonization.

Use of the above substance for treatment and/or prevention of diseases in the manufacture of medicaments, health products, food, and food additives.

The essence of health is the dynamic balance of the structure system, and the imbalance of the structure system is the overall source of diseases. In order to treat or prevent imbalance of the biological structure system, including the imbalance inside the biological structure system and the structural imbalance between the biological structure system and the external structure. The present disclosure allows the biological structure system to recognize the structure information of the stable structure corresponding to the substance, and uses the self-adapting and self-organizing function of the biological structure system to regulate its abstract structure (such as gene expression), and to produce or modulate the synthesis or secretion of structure(s) interacting with the substance, hereby treating or preventing diseases associated with the substance.

The self-adapting and self-organizing function of the biological structure system, that is, the function of establishing a balance between the biological structure system and the external structure by their interaction, is especially seen when the external structure imposes a great stress on the biological structure system, i.e. a large volume of information or a long term action from the external structure. For example, the drug resistance generated by bacteria is the result of the self-adaptability of its own structure, which produces a counteractive substance such that the organism can adapt to this substance, eliminate the harm, and even use it as its own. Bacterial resistance to drugs, also known as drug resistance, can be classified into inherent drug resistance and acquired drug resistance. Inherent drug resistance is also known as natural drug resistance, determined by bacterial chromosomal genes, and passed from generation to generation without a change. For example, streptococci are naturally resistant to aminoglycoside antibiotics. Acquired drug resistance is due to development of drug resistance in bacteria after contact with antibiotics by multiple mechanisms, such as: 1. Production of inactivating enzymes: production of inactivating enzymes in bacteria to inactivate antibacterial agents is one of the most important mechanisms of drug resistance generation; the enzymes destroy antibacterial agents and their antibacterial activity before they can act on the bacteria, and can be expressed by plasmids and chromosomal genes; the acquired drug resistance in bacteria may disappear if not in contact with antibiotics any further, and may be passed to the next generation by transfer of the drug resistance gene from plasmids to chromosome, becoming inherent drug resistance. 2. Change of the target site of the antibacterial agent: the target protein at the binding site of the antibiotic on the cell inner membrane is changed, and thus the affinity with the antibiotic is reduced, so that the antibiotic cannot bind to it and fails to kill the bacteria; for example, the high resistance of *Streptococcus pneumoniae* to penicillin is produced through this mechanism; after the bacteria come into contact with antibiotics, a new target protein that is not present in the original sensitive bacteria is produced, so that the antibiotic cannot bind to the new target protein, resulting in a high degree of resistance.

It can be seen from the above technical solutions that the mechanism of treating or preventing diseases with a substance according to the present disclosure, that is, the mechanism of treating diseases with the stable structure corresponding to a substance, mainly lies in introduction of structural information of the stable structure system from the external environment. The stable structure corresponding to a substance is obtained by high-temperature carbonization of the substance, in which case the active organic substance has been inactivated and causes no harm to the organism, and will not cause adverse reactions such as allergies and intolerance. Therefore, enough stress from external structure information can be applied to the organism to enable it to cross the threshold from a quantitative change to a qualitative change. Therefore, the present disclose does not rely on the absorption and metabolism of substances by an organism, nor on direct interference with the imbalance of the biological structure system by means of the substance, but mainly relies on the recognition of the stable structure corresponding to the substance by the organism, after which the organism uses its own self-adapting and self-organizing function to restore the balance of the biological structure system or establish a balance with the substance structure, and the substance for treating diseases (i.e. the stable structure corresponding to the substance) is excreted from the body mainly by the excretion system of the organism, to achieve the purpose of treating diseases associated with the substance.

A method for designing a substance for treatment and/or prevention of diseases, comprising:
using a stable structure corresponding to a substance as the substance for treatment and/or prevention of diseases caused by the substance.

The stable structure corresponding to the substance refers to a structure of the substance that is present in a solid state under a high temperature of 500°C or more.

A substance for treatment and/or prevention of diseases, designed according to the above method.

A method for preparing the above substance for treatment and/or prevention of diseases, wherein the stable structure corresponding to the substance or to a substance containing the substance is prepared by high-temperature carbonization.

Use of the above substance for treatment and/or prevention of diseases in the manufacture of medicaments, health products, food, and food additives.

A method for designing a substance for treatment and/or prevention of lactose intolerance, comprising: using a stable structure corresponding to lactose or to a substance containing lactose as the substance for treatment and/or prevention of lactose intolerance.

The stable structure corresponding to the substance refers to a structure of the substance that is present in a solid state under a high temperature of 500°C or more.

A substance for treatment and/or prevention of lactose intolerance, designed according to the above method.

A method for preparing the above substance for treatment and/or prevention of lactose intolerance, wherein the stable structure corresponding to lactose or to a substance containing lactose is prepared by high-temperature carbonization.

The substance containing lactose includes milk powder, cow milk, or the like that contains lactose.

Use of the above substance for treatment and/or prevention of lactose intolerance in the manufacture of medicaments, health products, food, and food additives.

The imbalance of the biological structure system includes the imbalance between the biological structure system itself and a non-self-structure. In the present disclosure, the biological structure system itself is called *"the positive (ZHENG, or GOOD)",* and the external structure that causes imbalance of the biological structure system is called *"the external negative (external XIE, or external BAD)". "The external negative"* is with respect to an organism, including substances harmful to a healthy organism, such as pathogenic bacteria and viruses, and also including substances that are harmless to a healthy organism, but in certain organisms induce onset of diseases upon contact, despite that there is no onset of diseases in the absence of the substances. For example, some substances are harmless or even beneficial to healthy organisms, indicating that the structure of these substances is in a balance with the structure of the organisms. However, if the structure of the organism is imbalanced, the structural balance between the substances and the organisms is broken, and various imbalances may cause different diseases, such as allergies or intolerance to these substances, etc., such as milk allergy and milk intolerance. Under the action of an external structure, with the self-adapting and self-organizing function of the biological structure system that maintains the structural stability, a structure that interacts with the external structure in an inhibitory or promoting manner will be generated, thereby establishing a balance between the biological structure system and the external structure, i.e. supporting the *"positive"* by the *"external negative".*

A method for designing a substance for treatment and/or prevention of diseases, comprising:
using a stable structure corresponding to a pathologically changed substance as the substance for treatment and/or prevention of diseases caused by a pathologically changed substance that is identical or similar to the pathologically changed substance.

The stable structure corresponding to the substance refers to a structure of the substance that is present in a solid state under a high temperature of 500°C or more.

A substance for treatment and/or prevention of diseases, designed according to the above method.

A method for preparing the above substance for treatment and/or prevention of diseases, wherein the stable structure corresponding to the pathologically changed substance or to a substance containing the pathologically changed substance is prepared by high-temperature carbonization.

A method for designing a substance for treatment and/or prevention of diseases, comprising:
using a stable structure corresponding to a pathologically unchanged substance as the substance for treatment and/or prevention of diseases caused by a pathological change in a substance that is identical or similar to the pathologically unchanged substance.

The stable structure corresponding to the substance refers to a structure of the substance that is present in a solid state under a high temperature of 500°C or more.

A substance for treatment and/or prevention of diseases, designed according to the above method.

A method for preparing the above substance for treatment and/or prevention of diseases, comprising: a stable structure corresponding to the pathologically unchanged substance or to a substance containing the pathologically unchanged substance is prepared by high-temperature carbonization.

Use of the above substance for treatment and/or prevention of diseases in the manufacture of medicaments, health products, food, and food additives.

The imbalance of the biological structure system includes the imbalance of the internal structure in the biological structure system, that is, the disease of the internal structure, which is often imbalance of structural interaction. For example, for a certain structure in the biological structure system, under-expression of genes of structures restricting it or over-expression of genes of structures promoting it will break the balance between structures. For example, tumor suppressor genes and proto-oncogenes restrict each other to maintain relative stability of positive and negative regulatory signals. When such genes are mutated, deleted or inactivated, the balance of interactions between structures is broken, which may cause malignant transformation of cells and lead to tumors. The present disclosure refers to the imbalanced structure in the biological structure system as *"the internal negative (internal XIE)".* According to the present disclosure, the stable structure corresponding to the imbalanced internal structure of an organism is used as an external structure to act on the biological structure system. Due to the self-adapting, self-organizing and self-stabilizing function of the biological structure system, the biological structure system will regulate the expression of genes of structures that interact with the external structure according to the structural information from the external structure, so as to achieve a balance with the external structure, thus realizing restoration of an internal balance for the imbalanced structure in the biological structure system and treatment of diseases, i.e. supporting the *"positive"* by the *"internal negative".*

For the imbalanced internal structures in the biological structure system, its corresponding balanced structures in each case has a structure interacting with it; and these balanced structures are also referred to as *"the positive"* in the present disclosure.

The stable structure corresponding to a balanced structure that is identical or similar to the imbalanced structure is used as an external structure to act on the biological structure system. Due to the self-adapting, self-organizing and self-stabilizing function of the biological structure system, the biological structure system will regulate the expression of genes of structures that interact with the external structure to a normal level according to the structural information from each structure in the external structure, so that the imbalanced structure restores the balance. For example, the stable structure corresponding to the swine bladder is used to treat human bladder diseases, such as frequent urination. According to the present disclosure, the stable structure corresponding to a pathologically unchanged substance is used as a substance for treatment and/or prevention of diseases caused by a pathological change in a substance identical or similar to the pathologically unchanged substance, i.e. supporting the *"positive"* by the *"positive".*

A method for designing a substance for treatment and/or prevention of diseases, comprising:
using a stable structure corresponding to substance C as the substance for treatment and/or prevention of disease A caused by a certain substance,
wherein
disease A is a disease caused by the certain substance;
substance B is a substance associated with disease A;
substance C is similar to or of the same type as substance B, and the certain substance does not induce disease A in an organism containing substance C.

The stable structure corresponding to the substance refers to the structure of the substance that is present in a solid state under a high temperature of 500°C or more.

A method for designing a substance for treatment and/or prevention of diseases, comprising:
using a stable structure corresponding to substance C as the substance for treatment and/or prevention of disease A,
wherein
disease A is the disease to be treated or prevented;
substance B is a substance associated with disease A;
substance C is similar to or of the same type as substance B, and an organism containing substance C would not have disease A.

The stable structure corresponding to the substance refers to the structure of the substance that is present in a solid state under a high temperature of 500°C or more.

A substance for treatment and/or prevention of diseases, designed according to the above method.

A method for preparing the above substance for treatment and/or prevention of diseases, comprising: the stable structure corresponding to substance C is prepared by high-temperature carbonization.

Use of the above substance for treatment and/or prevention of diseases in the manufacture of medicaments, health products, food, and food additives.

A method for designing a substance for treatment and/or prevention of rhinitis, comprising:
using a stable structure corresponding to a substance associated with rhinitis as the substance for treatment and/or prevention of rhinitis.

Preferably, the stable structure corresponding to the substance refers to a structure of the substance that is present in a solid state under a high temperature of 500°C or more.

Rhinitis is an inflammatory disease in the nasal cavity, which is an inflammation of the nasal mucosa caused by viruses, bacteria, allergens, various physical and chemical factors, and certain systemic diseases. Rhinitis caused by allergens is also called allergic rhinitis. As the course of the disease progresses, nasal mucosa congestion, swelling, exudation, hyperplasia, atrophy or necrosis can develop into chronic rhinitis. Therefore, from the perspective of Western medicine, the allergens that cause rhinitis are substances associated with the disease. From the structural point of view, the root cause is the imbalance due to interactions between the biological structure system and the structure of the allergen. From the perspective of traditional Chinese medicine, "the lung opens up at the nose", and the lung communicates with the nature through the skin and hair in the nose and the mouth, and easily senses the invasion of foreign cold wind and air. In spring, there are many external winds that easily invade the head, face and skin surface. If the lung is weak at this time, the lung defense is compromised and the skin and muscle are relaxed, the wind often penetrates through the mouth and nose or the skin and hair to impede vascular circulation and cause blockage feelings, and the nasal orifices lose their patency and nourishment and become ill. If the lung is weak, the cold wind and "external negatives" can invade and cause nasal congestion, sneezing, and runny nose. If the wind enters from the nose and mouth, it will cause nasal congestion, runny nose and sticky nose. Therefore, according to the theory of traditional Chinese medicine, the cause of rhinitis is ascribed to the lungs. From a structural point of view, the root cause is the imbalance of some structures in the structure system of the lung of an organism, which leads to the structural imbalance between the biological structure system and the structure of the allergen. The balance of the biological structure system is dynamic and is associated with the external structural environment. For example, for humans, some substances, such as *Artemisia desertorum,* easily cause universal allergic rhinitis in humans, which means that the structural imbalance of the lung is a relative concept. For example, organisms such as swine, bovine, and Caprinae are not allergic to *Artemisia desertorum* and will not have allergic rhinitis. That is, the lung of swine, bovine and Caprinae contain structures that interact with *Artemisia desertorum* to avoid allergic rhinitis, i.e., structures that inhibit rhinitis.

Preferably, the stable structure corresponding to a substance associated with rhinitis is one or more selected from:

### (1) A stable structure corresponding to a substance causing rhinitis in an organism;

The imbalance of the biological structure system includes the imbalance between the biological structure system itself and a non-self structure. In the present disclosure, the biological structure system itself is called *"the positive",* and the external structure that causes imbalance of the biological structure system is called *"the external negative".* Substances causing rhinitis in an organism are *"the external negative",* and will result in structural imbalance causing symptoms of allergic rhinitis in the organism when acting on the biological structure system. The essence is that the biological structure system having allergic rhinitis fails to reach a dynamic balance with *"the external negative".* According to the present disclosure, the stable structure corresponding to *"the external negative"* is used as an external structure to act on the biological structure system. Due to the self-adapting, self-organizing and self-stabilizing function of the biological structure system, the biological structure system will regulate the expression of genes of structures that interact with an external structure according to the structural information from the external structure, so as to achieve a balance with the external structure, thereby establishing a balance between the biological structure system and *"the external negative",* i.e. supporting the *"positive"* by the *"external negative".*

### (2) A stable structure corresponding to a rhinitis-associated substance of an organism itself;

The imbalance of the biological structure system includes the imbalance of the internal structure in the biological structure system, that is, the disease of the internal structure, which is often the imbalance of structural interaction. For example, for a certain structure in the biological structure system, under-expression of genes of structures restricting it or over-expression of genes of structures promoting it will break the balance between structures. The present disclosure refers to the imbalanced structure in the biological structure system as *"the internal negative".* Rhinitis is associated with an imbalanced structure in the lung, that is, the imbalanced structure in the lung that causes rhinitis is *"the internal negative",* a rhinitis-associated substance of the organism itself. The stable structure corresponding to *"the internal negative"* is used as an external structure to act on the biological structure system. Due to the self-adapting, self-organizing and self-stabilizing function of the biological structure system, the biological structure system will regulate the expression of genes of structures that interact with the external structure according to the structural information from the external structure, so as to achieve a balance with the external structure, thus realizing restoration of a balance of the imbalanced internal structure in the biological structure system and treatment of diseases, i.e. supporting the *"positive"* by the *"internal negative".*

For the imbalanced internal structures in the biological structure system, its corresponding balanced structures in each case has a structure interacting with it; and these balanced structures are also referred to as *"the positive"* in the present disclosure.

Rhinitis is associated with an imbalance in the structures of the lungs, which are " *internal negative* ". A lung from healthy organism without rhinitis is a *"positive",* and its corresponding stable structure is used as an external structure to act on the biological structure system. Due to the self-adapting, self-organizing and self-stabilizing function of the biological structure system, the biological structure system will regulate the expression of genes of structures that interact with the external structure to a normal level according to the structural information from each structure in the external structure, so that the imbalanced structure in the biological structure system restores the balance, i.e. supporting the *"positive"* by the *"positive".*

### (3) A stable structure corresponding to a substance containing a rhinitis-inhibiting structure;

If a substance capable of causing a disease does not cause the disease in a certain organism, then the structure system of the certain organism can achieve a balance with the structure of the disease-causing substance upon interaction, that is to say, the disease-associated structure in the certain organism has a structure that interacts with the structure of the disease-causing substance and prevents the disease in the organism, and such a structure is not isolated but in interactions with other structures in the biological structure system, thereby reaching a balance. Some substance generally causes allergic rhinitis in human, but not in certain organisms such as swine, bovine and Caprinae. Therefore, the stable structure corresponding to the lung of the organism in which allergic rhinitis would not be caused is used as an external structure to act on the biological structure system in need of treatment of this disease. Due to the self-adapting, self-organizing and self-stabilizing function of the biological structure system, the biological structure system will regulate the genes or expression of genes of structures that interact with the external structure according to the structural information from each structure in the external structure, so that a balance is achieved between the biological structure system and the structure of the disease-causing substance, which is also supporting the *"positive"* by the *"positive".*

### (4) A stable structure corresponding to a substance containing the substance according to any one of (1) to (3) above.

In another preferred embodiment, the rhinitis-associated substance is derived from the lung of an organism.

A substance for treatment and/or prevention of rhinitis, designed according to the above method.

A method for preparing the above substance for treatment and/or prevention of rhinitis, wherein the stable structure corresponding to the substance is prepared by high-temperature carbonization.

The substance for treatment and/or prevention of rhinitis is prepared by high-temperature carbonization of a substance containing a rhinitis-associated substance, because such a substance is easily available and makes the problem simpler. For allergens that externally cause allergic rhinitis, allergen-containing substances are used directly without the need to isolate the specific allergens or the need to know the specific mechanism of allergies in an organism. For instance, for allergic rhinitis caused by pollen, there is no need to know or isolate the specific allergy-causing substances in the pollen, and the pollen is directly subjected to high-temperature carbonization to make a desensitizing substance for treatment and/or prevention of rhinitis, so as to support the *"positive"* with the *"external negative".* Following the same principle, the lung, as a rhinitis-associated substance of an organism, preferably of swine, bovine, Caprinae or primate, may be directly prepared into a substance for treatment and/or prevention of rhinitis by high-temperature carbonization, so as to support the *"positive"* with the *"external negative".*

Use of the above substance for treatment and/or prevention of rhinitis in the manufacture of medicaments, health products, food, and food additives.

If a substance capable of causing a disease does not cause the disease in a certain organism, then the structure system of the certain organism can achieve a balance with the structure of the disease-causing substance upon interaction, that is to say, the disease-associated structure in the certain organism has a structure that interacts with the structure of the disease-causing substance and prevents the disease in the organism, and such a structure is not isolated but in interactions with other structures in the biological structure system, thereby reaching a balance. The stable structure corresponding to the disease-associated substance of the organism is used as an external structure to act on the biological structure system in need of treatment of this disease. Due to the self-adapting, self-organizing and self-stabilizing function of the biological structure system, the biological structure system will regulate the genes or expression of genes of structures that interact with the external structure according to the structural information from each structure in the external structure, so that a balance is achieved between the biological structure system and the structure of the disease-causing substance, which is also supporting the *"positive"* by the *"positive".*

A culturing method for preparing a substance for treatment and/or prevention of diseases, comprising: using a substance associated with the diseases to be treated and/or prevented to culture a substance for treatment and/or prevention of diseases.

Preferably, the using a substance associated with the diseases to be treated and/or prevented to culture a substance for treatment and/or prevention of diseases comprises: developing the tolerance of the organism with the substance that directly causes the disease, until the disease-causing substance does not cause the disease in the organism; and
using a substance of the organism or of an offspring of the organism as the substance for treatment and/or prevention of diseases.

The organism is preferably swine, bovine, Caprinae, primate, and the like.

Preferably, the using a substance associated with the diseases to be treated and/or prevented to culture a substance for treatment and/or prevention of diseases comprises: cultivating an organism with the stable structure corresponding to the substance associated with the diseases, until the organism will not have the disease; and using a substance of the organism or an offspring of the organism as the substance for treatment and/or prevention of diseases.

The organism is preferably swine, bovine, Caprinae, primate, and the like.

The stable structure corresponding to the substance refers to a structure of the substance that is present in a solid state under a high temperature of 500°C or more.

In the present disclosure, the organism may be the same or similar species, preferably swine, bovine, Caprinae, or primate.

The essence of health is the dynamic balance of the structure system. This dynamic balance is the result of the interactions between structures in the structure system. The most basic interactions are promotion and restriction. In a biological structure system, if a structure does not have another structure promoting it, this structure will be deficient, and if it does not have another structure restricting it, this structure will be excessive, both cases resulting in imbalance in the structure system. The essence of a disease is imbalance of the biological structure system. It can be seen from the above technical solutions that the method for treating or preventing diseases according to the present disclosure uses the stable structure of a disease-associated substance as an external structure to act on the biological structure system (such as by oral administration), and the biological structure system regulates genes or expression of genes by recognition and transmission of the structural information from the external structure and by its self-adapting and self-organizing function, so as to treat or prevent diseases associated with the substance.

It can be seen from the above technical solutions that the substance for treatment and/or prevention of diseases, the method for designing the substance, and the method for preparing the substance according to the present disclosure have the following beneficial effects.
1. The methods for designing and preparing the substance for treatment and/or prevention of diseases according to the present disclosure do not focus on the specific structure of the disease-causing substance or the specific mechanism of pathogenicity, but on using the stable structure corresponding to an external disease-causing substance or internal substance, or a substance identical or similar to the disease-causing external or internal substance as an external structure to act on the biological structure system. The external structure can make the organism to eliminate, alleviate, or prevent the diseases by self-adaption and self-organization of structures, thereby being universally applicable.
2. For diseases caused by an external substance, there is no need to investigate which specific components in the external substance cause the diseases, and it is sufficient to use only the substance containing the external substance. For example, for peanut allergy, there is no need to investigate what substances in peanut are allergens, and using only peanut is sufficient. For diseases caused by internal pathogenesis of an organism, one only needs to know the specific pathologically changed cells, tissue or organs to allow use of the cells, tissue or organs in the same or similar species, without the need to investigate the mechanism of pathogenicity or the specific symptoms of the diseases. What is needed is to use the stable structure corresponding to the above-mentioned disease-causing substance as an external structure to act on the biological structure system, and then the biological structure system can establish a balance with the structure of the external substance or restore the internal balance in the biological structure system according to self-adaptation and self-organization of structures, thereby eliminate, alleviate, or prevent diseases. For example, the stable structure corresponding to peanut is used to to treat and/or prevent peanut allergy or peanut intolerance; and the stable structure corresponding to the lung of swine is used to treat and/or prevent asthma or lung-associated diseases such as allergic rhinitis.
3. The substance for treatment and/or prevention of diseases, the method for designing the substance, and the method for preparing the substance according to the present disclosure circumvent the need to study the disease mechanism, greatly save time, manpower, material resources, and financial resources, significantly shorten the drug R&D cycle, and lower the cost of treatment of diseases, representing an extreme simplification of drug research and development, and diagnosis and treatment of diseases.

### Detailed description of invention

The present disclosure relates to a substance for treatment and/or prevention of diseases, the method for designing the substance, and the method for preparing the substance. Those skilled in the art can learn from the content of the specification to use different materials or improve the process or use other similar process for preparation, which are all considered to be included in the scope of the present disclosure. It is particularly noteworthy that all similar replacements and modifications are obvious to those skilled in the art, and they are all deemed to be included in the scope of the present disclosure. The method and product of the present disclosure have been described with reference to preferred examples, and it is obvious that those skilled in the art can make modifications or appropriate changes and combinations to the methods described herein without departing from the content, spirit and scope of the present disclosure to carry out and apply the technique of the present disclosure.

In order to provide further understanding of the present disclosure, the technical solutions in examples of the present disclosure will be clearly and completely described below in conjunction with the examples of the present disclosure. Apparently, the examples described below are only a part of examples of the present invention rather than all of them. Based on the examples of the present disclosure, all other examples obtained by those of ordinary skill in the art without paying creative work shall fall within the protection scope of the present invention.

The embodiments are as follows.

### Example 1 Substance for treatment and/or prevention of lactose intolerance, method for designing the substance, and method for preparing the substance

Lactose intolerance in an organism is due to imbalance between the biological structure system and the structure of lactose. The stable structure corresponding to lactose was used as the external structure to act on a lactose intolerant organism, and the biological structure system established a balance between the biological structure system and the structure of lactose under the action of the external structure. Therefore, using the stable structure corresponding to lactose as the substance for treating and/or preventing lactose intolerance represents the method for designing and preparing the substance for treatment and/or prevention of diseases in an organism directly caused by foreign obj ects.

The stable structure corresponding to the substance is characterized by the structure of the substance that is present in a solid state under a high temperature of 500°C or more. Therefore, in the method for preparing the substance for treatment and/or prevention of lactose intolerance, the stable structure corresponding to lactose or a lactose-containing substance can be prepared by high-temperature carbonization. Because milk is easier to obtain than pure lactose, the stable structure corresponding to milk powder containing lactose was prepared by high-temperature carbonization.

### Example 2 Clinical study with the substance prepared in Example 1 in patients with lactose intolerance

This example used the substance prepared in Example 1 for efficacy verification, as specifically shown below.
1. Clinical data: A total of 38 cases of outpatients and inpatients with lactose intolerant were enrolled, including 18 males and 20 females, aged from 2 to 13 years old. The symptoms were mainly gastrointestinal discomfort (including various degrees of abdominal pain, constipation or diarrhea, distended tummy, fetid breath, etc.).
2. Diagnosis criteria: According to the patients' medical history, a double-blind placebo-controlled food provocation test was conducted as the diagnostic criteria for testing food intolerance to diagnose lactose intolerance.
3. Treatment method: The patients orally took the substance prepared in Example 1, 3 g per time, once a day, for 3 consecutive days.
4. Efficacy evaluation criteria: "cured" means regression of clinical symptoms, formed stools, and normal microscopy; "effective" means substantial regression of clinical symptoms, changed stool components, reduced frequency of stools, and a small number of red blood cells or white blood cells detectable in microscopy of stools; "ineffective" means no apparent improvement in clinical symptoms or even aggravated symptoms, unchanged stool components, and watery stools. Total effective rate = cured rate + effective rate.
5. Efficacy results: After treatment, 35 patients were cured, 2 patients showed "effective", and one patient showed "ineffective", with a total effective rate of 97.37%. After treatment, these patients consumed different dairy products such as cow milk several times and did not have any symptoms. And during the efficacy observation period, no side effect or drug dependence was observed.

### Example 3 Substance for treatment and/or prevention of ankylosing spondylitis, method for designing the substance, and method for preparing the substance

Ankylosing spondylitis is a chronic inflammatory disease whose main symptoms are sacroiliitis and spinal enthesitis, characterized by fibrosis and ossification of the large joints of the limbs, the annulus fibrosus disci intervertebralis and its nearby connective tissue, as well as ankylosis. Therefore, from the structural point of view, needed is use of the stable structure corresponding to the connective tissue as an external structure to act on the organism suffering from ankylosing spondylitis to restore the balance of the structure system. Therefore, using the stable structure corresponding to the connective tissue as a substance for treatment and/or prevention of ankylosing spondylitis represents the designing and preparation methods in which the stable structure corresponding to a pathologically unchanged substance is used as the substance for treatment and/or prevention of diseases caused by a pathological change in a substance identical or similar to the pathologically unchanged substance, i.e. supporting *"the positive"* with *"the positive".* Because the connective tissue of bovine is easily available, in this example, the connective tissue of healthy bovine was subjected to high-temperature carbonization to obtain the substance for treatment or prevention of ankylosing spondylitis.

### Example 4: Clinical study with the substance prepared in Example 3 in patients with ankylosing spondylitis

1. Clinical data: This example enrolled totally 54 patients with ankylosing spondylitis, who were randomly divided into the treatment group of 28 patients and a control group of 26 patients according to the order of visit. The treatment group comprised 23 males and 5 females, aged from 31 to 52 years old, with an average course of disease of 10.3 years. The control group comprised 22 males and 4 females, aged from 33 to 51 years old, with an average course of disease of 10.1 years. There was no statistically significant difference between the two groups of patients in gender, age, condition and other clinical data (P>0.05), and they were comparable.
2. Diagnosis criteria: (1) Unexplained lumbago and leg pain/discomfort that occurred before the age of 40; (2) Insidious onset; (3) The course of disease >1 week; (4) Morning stiffness, static, or night pain, alleviated after exercise; (5) Positive for Patrick sign test (Fabere test), percussion pain in sacroiliac joints and sacrum; (6) Inflammatory changes in the imaging examination of sacroiliac joints. Ankylosing spondylitis can be considered if one of the clinical criteria (1) to (4) is met on the basis of (5); and ankylosing spondylitis can be diagnosed if one of the clinical criteria is met on the basis of (6).
3. Treatment method: All patients were given diet and functional exercise guidance. Patients in the treatment group orally took the substance prepared in Example 3, 3 g per time, once a day, for 7 days. Patients in the control group orally took sulfasalazine enteric tablets, 3 tablets per time, 2 times a day, for 1 month.
4. Efficacy evaluation criteria: The total effective rate = markedly effective rate + effective rate. "Markedly effective" means that the patient's clinical symptoms and signs are significantly alleviated after treatment; "effective" means that the patient's clinical symptoms and signs are improved after treatment, but not to the standard level of "markedly effective"; "ineffective" means that the patient's clinical symptoms or signs are not improved after treatment or even become severe.
5. Treatment results: Both groups were treated for 1 month and observed for efficacy. Among the 28 patients in the treatment group, 24 showed "markedly effective", 3 showed "effective", and one showed "ineffective", with a total effective rate of 96.42%. Among the 26 patients in the control group, 4 cases showed "markedly effective", 10 showed "effective", and 12 showed "ineffective", with a total effective rate of 53.85%. The difference in the total effective rate between the treatment group and the control group was statistically significant (P<0.001), that is, the treatment group showed significantly better efficacy than the control group (see Table 1). During the treatment, patients in the treatment group did not show adverse drug reaction or drug dependence.

**Table 1 Comparison of the effective rates between the two groups after treatment (Example)**

| Group | Number of patients | Markedly effective | Effective | Ineffective | Total effective rate (%) |
|---|---|---|---|---|---|
| Treatment | 28 | 24 | 3 | 1 | 96.42% |
| Control | 26 | 4 | 10 | 12 | 53.85% |

### Example 5 Substance for treatment and/or prevention of frequent urination, method for designing the substance, and method for preparing the substance

When there is inflammation, an injury or a disease in the bladder, its elasticity decreases, the urine storage capacity is reduced, the nerve perception threshold is lowered, and the urinary central nerve is in an excited state, then frequent urination is caused. The structural imbalance of the bladder causes functional imbalance and frequent urination. A bladder with a balanced structure is described as *"the positive"* in the present disclosure. Each structure is in interaction with another structure. If *"the positive"* is used as an external structure to act on the biological structure system, due to the self-adapting, self-organizing and self-stabilizing function of the biological structure system, the biological structure system will regulate the expression of genes of structures that interact with the external structure to a normal level according to the structural information from each structure in the external structure, so that the imbalanced structure restores the balance. Therefore, the stable structure corresponding to the bladder of an animal like swine, bovine, or Caprinae can be used to treat frequent urination in a human, which represents the designing and preparation methods in which the stable structure corresponding to a pathologically unchanged substance is used as a substance for treatment and/or prevention of diseases caused by a pathological change in a substance identical or similar to the pathologically unchanged substance, i.e. supporting the *"positive"* by the *"positive".*

In this example, a bladder of healthy swine was selected and subjected to high-temperature carbonization to obtain the substance for treatment or prevention of frequent urination.

### Example 6: Clinical study with the substance prepared in Example 5 in patients with frequent urination

1. Clinical data: 33 cases (a total number) of outpatients and inpatients with frequent urination were enrolled, and a before-and-after study was conducted.
2. Diagnosis criteria: for urinating 4-6 times during the day and 0-2 times at night, significantly increased frequency is regarded as frequent urination. Patients with frequent urination due to a non-bladder disorder were excluded.
3. Treatment method: The patients orally took the substance for treating frequent urination prepared in Example 5, 3 g per time, once a day, for 14 consecutive days.
4. Efficacy evaluation criteria: "cured" means that the frequency of urination returns to normal; "effective" means that the frequency of urination is significantly reduced; "ineffective" means that the frequency of urination is not significantly reduced or even increased.
5. Treatment results: The effectiveness was observed after treatment. Among 33 patients, 15 showed markedly effective, 16 showed effective, and 2 showed ineffective, with a total effective rate of 93.94%.

### Example 7 Substance for treatment and/or prevention of allergic rhinitis, method for designing the substance, and method for preparing the substance

Rhinitis is an inflammatory disease in the nasal cavity, which is an inflammation of the nasal mucosa caused by viruses, bacteria, allergens, various physical and chemical factors, and certain systemic diseases. Rhinitis caused by allergens is also called allergic rhinitis. As the course of the disease progresses, nasal mucosa congestion, swelling, exudation, hyperplasia, atrophy or necrosis can develop into chronic rhinitis. Therefore, from the perspective of Western medicine, the allergen that causes rhinitis includes substances associated with the disease. From the structural point of view, the root cause is the imbalance due to interactions between the biological structure system and the structure of the allergen. From the perspective of traditional Chinese medicine, "the lung opens up at the nose", and the lung communicates with the nature through the skin and hair in the nose and the mouth, and easily senses the invasion of foreign cold wind and air. In spring, there are many external winds that easily invade the head, face and skin surface. If the lung is weak at this time, the lung defense is compromised and the striae of the skin and muscle is loose, the wind often penetrates through the mouth and nose or the skin and hair to impede vascular circulation and cause blockage feelings, and the nasal orifices lose their patency and nourishment and become ill. If the lung is weak, the cold wind and "external negatives" can invade and cause nasal congestion, sneezing, and runny nose. If the wind enters from the nose and mouth, it will cause nasal congestion, runny nose and sticky nose. Therefore, according to the theory of traditional Chinese medicine, the cause of rhinitis is ascribed to the lungs. From a structural point of view, the root cause is the imbalance of some structures in the structure system of the lung of an organism, which leads to the structural imbalance between the biological structure system and the structure of the allergen. The balance of the biological structure system is dynamic and is associated with the external structural environment. For example, for humans, some substances, such as *Artemisia desertorum,* easily cause universal allergic rhinitis in humans, which means that the structural imbalance of the lung is a relative concept. For example, organisms such as swine, bovine, and Caprinae are not allergic to *Artemisia desertorum* and will not have allergic rhinitis. That is, the lung of swine, bovine and Caprinae contain structures that interact with *Artemisia desertorum* to avoid allergic rhinitis, i.e., structures that inhibit rhinitis.

The lung of an organism contains substances associated with allergic rhinitis and is readily available. Therefore, in this example, the lung of swine was selected and subjected to high-temperature carbonization to obtain the substance for treatment or prevention of allergic rhinitis.

### Example 8 Clinical application trial with the substance prepared in Example 7 for treatment of allergic rhinitis

This example used the substance prepared in Example 7 above for efficacy verification, as specifically shown below.
1. Clinical data: 60 cases (a total number) of outpatients and inpatients with allergic rhinitis were enrolled, and were randomly divided into a treatment group with 30 patients and a control group with 30 patients. There was no significant difference between the two groups in clinical data and conditions. There was no statistically significant difference between the two groups in age, gender, course of disease, and complication (P>0.05), and they were comparable.
2. Diagnosis criteria: 2 or more clinical symptoms such as sneezing, clear water-like nasal discharge, nasal congestion, and nasal itching, appearing continuously or continually for 1 hour or more every day. It may be accompanied by eye symptoms such as eye itching and conjunctival hyperemia. Common signs often include pale nasal mucosa, edema, and watery nasal discharge. Positive in the allergen skin prick test.
3. Treatment method: the patients in the treatment group orally took the substance associated with treatment of allergic rhinitis prepared in Example 7, 3 g per time, once a day, for 3 consecutive days. The patients in the control group orally took loratadine 10 mg per time, once day, for 30 days.
4. Efficacy evaluation criteria: "cured" means that symptoms and signs disappear for 60 days without recurrence; "effective" means that symptoms and signs are alleviated, and the number of onsets is reduced; "ineffective" means that the effective standard is not met. Total effective rate = cured rate + effective rate.
5. Efficacy results: Table 2 shows that there were totally 28 patients in the treatment group showing "cured" and "effective" after treatment, with a total effective rate of 93.33%. In contrast, the control group showed a total effective rate of 66.67% after treatment, which is significantly lower (P<0.05). During the treatment, patients in the treatment group experienced no drug adverse reaction or drug dependence, and no recurrence during the 6-month follow-up, while adverse reactions occurred in 5 cases from the control group and allergic symptoms appeared after contact with allergens after drug discontinuance.

**Table 2 Comparison of effectiveness of treatment of patients with allergic rhinitis (as Examples)**

| .Group | Number of patients | Cured | Effective | Ineffective | Total effective rate (%) |
|---|---|---|---|---|---|
| Treatment | 30 | 22 | 6 | 2 | 93.33% |
| Control | 30 | 0 | 20 | 10 | 66.67% |

## Claims

1. A method for designing a substance for treatment and/or prevention of diseases, comprising:
using a stable structure corresponding to a substance as the substance for treatment and/or prevention of diseases associated with the substance;
wherein the stable structure corresponding to the substance refers to a structure of the substance that is present in a solid state under a high temperature of 500°C or more.

2. The method of claim 1, wherein the diseases associated with the substance include a disease caused by some materials in the substance and/or a disease caused by changes in some materials in the substance.

3. A substance for treatment and/or prevention of diseases, designed according to the method of claim 1 or 2.

4. A method for preparing the substance for treatment and/or prevention of diseases of claim 3, wherein the stable structure corresponding to the substance is prepared by high-temperature carbonization.

5. A method for designing a substance for treatment and/or prevention of diseases, comprising:
using a stable structure corresponding to a substance as the substance for treatment and/or prevention of diseases caused by the substance;
wherein the stable structure corresponding to the substance refers to a structure of the substance that is present in a solid state under a high temperature of 500°C or more.

6. A substance for treatment and/or prevention of diseases, designed according to the method of claim 5.

7. A method for preparing the substance for treatment and/or prevention of diseases of claim 6, wherein the stable structure corresponding to the substance is prepared by high-temperature carbonization.

8. A method for designing a substance for treatment and/or prevention of lactose intolerance, comprising:
using a stable structure corresponding to lactose or to a lactose-containing substance as the substance for treatment and/or prevention of lactose intolerance;
wherein the stable structure corresponding to the substance refers to a structure of the substance that is present in a solid state under a high temperature of 500°C or more.

9. A substance for treatment and/or prevention of lactose intolerance, designed according to the method of claim 8.

10. A method for preparing the substance for treatment and/or prevention of lactose intolerance of claim 9, wherein the stable structure corresponding to lactose or a lactose-containing substance is prepared by high-temperature carbonization.

11. A method for designing a substance for treatment and/or prevention of diseases, comprising:
using a stable structure corresponding to a pathologically changed substance as the substance for treatment and/or prevention of diseases caused by a pathologically changed substance identical or similar to the pathologically changed substance;
wherein the stable structure corresponding to the substance refers to a structure of the substance that is present in a solid state under a high temperature of 500°C or more.

12. A substance for treatment and/or prevention of diseases, designed according to the method of claim 11.

13. A method for preparing the substance for treatment and/or prevention of diseases of claim 12, wherein the stable structure corresponding to the pathologically changed substance or to a substance containing the pathologically changed substance is prepared by high-temperature carbonization.

14. A method for designing a substance for treatment and/or prevention of diseases, comprising:
using a stable structure corresponding to a pathologically unchanged substance as the substance for treatment and/or prevention of diseases caused by a pathological change in a substance identical or similar to the pathologically unchanged substance;
wherein the stable structure corresponding to the substance refers to a structure of the substance that is present in a solid state under a high temperature of 500°C or more.

15. A substance for treatment and/or prevention of diseases, designed according to the method of claim 14.

16. A method for preparing the substance for treatment and/or prevention of diseases of claim 15, comprising: a stable structure corresponding to the pathologically unchanged substance or to a substance containing the pathologically unchanged substance is prepared by high-temperature carbonization.

17. A method for designing a substance for treatment and/or prevention of diseases, comprising,
using a stable structure corresponding to substance C or a substance containing substance C as the substance for treatment and/or prevention of disease A caused by a certain substance,
wherein
disease A is a disease caused directly by the certain substance or caused by a pathological change in the certain substance;
substance B is a substance associated with disease A;
substance C is similar to or of the same type as substance B, and the certain substance does not induce disease A in an organism containing substance C;
the stable structure corresponding to the substance refers to a structure of the substance that is present in a solid state under a high temperature of 500°C or more.

18. A method for designing a substance for treatment and/or prevention of diseases, comprising:
using a stable structure corresponding to substance C or a substance containing substance C as the substance for treatment and/or prevention of disease A,
wherein
disease A is the disease to be treated or prevented;
substance B is a substance associated with disease A;
substance C is similar to or of the same type as substance B, and an organism containing substance C would not have disease A;
the stable structure corresponding to the substance refers to a structure of the substance that is present in a solid state under a high temperature of 500°C or more.

19. A substance for treatment and/or prevention of diseases, designed according to the method of claim 17 or 18.

20. A method for preparing the substance for treatment and/or prevention of diseases of claim 19, comprising: the stable structure corresponding to substance C or a substance containing substance C is prepared by high-temperature carbonization.

21. A method for designing a substance for treatment and/or prevention of rhinitis, comprising:
using a stable structure corresponding to a substance associated with rhinitis as the substance for treatment and/or prevention of rhinitis;
wherein the stable structure corresponding to the substance refers to a structure of the substance that is present in a solid state under a high temperature of 500°C or more.

22. The method of claim 21, wherein the stable structure corresponding to the substance associated with rhinitis is one or more selected from:
(1) a stable structure corresponding to a substance causing rhinitis in an organism;
(2) a stable structure corresponding to a rhinitis-associated substance of an organism itself;
(3) a stable structure corresponding to a substance containing a rhinitis-inhibiting structure; and
(4) a stable structure corresponding to a substance containing the substance according to any one of (1) to (3) above.

23. A substance for treatment and/or prevention of rhinitis, designed according to the method of claim 21 or 22.

24. A method for preparing the substance for treatment and/or prevention of rhinitis of claim 23, wherein the stable structure corresponding to a substance associated with rhinitis is prepared by high-temperature carbonization.

25. Use of the substance of any one of claims 3, 6, 9, 12, 15, 19 and 23 in the manufacture of medicaments, health products, food, and food additives.

26. A culturing method for preparing a substance for treatment and/or prevention of diseases, comprising: using a substance associated with the diseases to be treated and/or prevented to culture a substance for treatment and/or prevention of diseases.

27. The method of claim 26, wherein the using a substance associated with the diseases to be treated and/or prevented to culture a substance for treatment and/or prevention of diseases comprises:
developing the tolerance of an organism with the substance that directly causes the disease, until the disease-causing substance does not cause the disease in the organism; and
using a substance of the organism or of an offspring of the organism as the substance for treatment and/or prevention of diseases;
wherein the organism is preferably swine, bovine, Caprinae, or primate.

28. The method of claim 26, wherein the using a substance associated with the diseases to be treated and/or prevented to culture a substance for treatment and/or prevention of diseases comprises:
cultivating an organism with the stable structure corresponding to the substance associated with the diseases, until the organism will not have the disease; and
using a substance of the organism or an offspring of the organism as the substance for treatment and/or prevention of diseases;
wherein the organism is preferably swine, bovine, Caprinae, or primate;
wherein the stable structure corresponding to the substance refers to a structure of the substance that is present in a solid state under a high temperature of 500°C or more.
